(19) Europäisches Patentamt European Patent Office Office européen des brevets

(11) **EP 3 733 174 A2**

(12) **EUROPEAN PATENT APPLICATION**
published in accordance with Art. 153(4) EPC

(43) Date of publication:
**04.11.2020 Bulletin 2020/45**

(51) Int Cl.:
***A61K 31/405*** (2006.01)    ***A61P 29/00*** (2006.01)

(21) Application number: **18897743.3**

(22) Date of filing: **27.12.2018**

(86) International application number:
**PCT/RU2018/000884**

(87) International publication number:
**WO 2019/132729 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2017 RU 2017146694**

(71) Applicant: **OBSHESTVO S OGRANICHENNOI OTVETSTVENNOSTJU "FORBOS" ("FORBOS") Moscow, 143026 (RU)**

(72) Inventors:
• **SCHWARZ, Joseph**
  **Toronto, 2 4 9 (CA)**
• **WEISSPAPIR, Michael**
  **Toronto, 3 4 1 (CA)**
• **SCHMYKOVA, Natalya Anatolyevna**
  **Tomsk, 634021 (RU)**
• **STANKEVICH, Sergey Aleksandrovich**
  **Tomsk, 634062 (RU)**
• **KHAZANOV, Veniamin Abramovich**
  **Tomsk, 634034 (RU)**

(74) Representative: **Jeck, Anton**
  **Jeck, Fleck & partner mbB**
  **Patentanwälte**
  **Klingengasse 2**
  **71665 Vaihingen/Enz (DE)**

(54) **INDOMETHACIN MENTHYL ESTER-BASED PHARMACEUTICAL COMPOSITIONS AND USE THEREOF**

(57)    The invention relates to pharmaceutical compositions based on a menthyl ester of indomethacin, which may be used as anti-inflammatory drugs to treat acute and chronic inflammatory diseases. The aforementioned pharmaceutical compositions have anti-inflammatory, analgesic and antipyretic action and don't have adverse ulcerogenic action in therapeutic doses. 1 independent claim, 7 tables, 1 figure, 13 examples.

EP 3 733 174 A2

## Description

### Field of the invention

The invention relates to medicine and anti-inflammatory drugs, in particular, to pharmaceutical compositions based on an indomethacin ester, which may be used to treat acute and chronic inflammatory diseases.

### Description of the prior art

The closest compound to the disclosed compound, a prototype of the invention, is indomethacin (1-(4-chlorobenzoyl)-5-methoxy-2-methyl-1H-indole-3-acetic acid), a well-known non-steroidal anti-inflammatory drug (NSAID), which exerts potent anti-inflammatory, analgesic and antipyretic action. Indomethacin is widely used in the form of capsules, tablets, suppositories, injections and other dosage forms of systemic action for treating arthritis and other inflammatory and degenerative diseases of musculoskeletal system, for treating pain of different causes, in combination therapy of some infectious-inflammatory and oncologic diseases.

However, indomethacin has high toxicity (LD50 for rats is less than 15 mg/kg in oral administration) and induces serious adverse effects in systemic use. In particular, indomethacin exerts the so-called ulcerogenic action, severely irritating the mucosal membranes of gastrointestinal tract (GI tract) organs, causing ulceration and dangerous bleeding. This ulcerogenic action is directly connected to the mechanism of action of indomethacin and other NSAIDs. Indomethacin is an inhibitor of both forms of the cyclooxygenase enzyme (COX-1 and COX-2), which are responsible for prostaglandin synthesis. When indomethacin reaches certain concentrations in the blood, prostaglandin synthesis becomes disrupted both at the inflammation site and in gastric mucosa, leading to erosion and ulcers.

Different indomethacin derivatives were studied to reduce the gastrointestinal toxicity of indomethacin. Different esters, amides, Mannich bases and some other hydrolysable compounds were synthesized. Such modification in some cases reduced the ulcerogenic effect or changed the selectivity of cyclooxygenase inhibition [1; 2]. Patent [3] discloses the use of a pharmaceutical composition containing a menthyl ester of indomethacin (MEI) as an ophthalmic drug for topical use for treating inflammatory eye diseases. Article [4] also describes the synthesis of several indomethacin derivatives, including its menthyl ester, and their use as NSAIDs. MEI efficacy is associated with its hydrolysis in the organism and the production of free indomethacin which has potent anti-inflammatory action. However, currently there are no drugs with systemic action based on indomethacin derivatives, including MEI, which would combine high anti-inflammatory activity and safety of use, in particular, low gastrointestinal toxicity. One of the reasons for this is the relationship between the potency of anti-inflammatory action and the severity of adverse ulcerogenic action. In case of MEI there is a second problem - low bioavailability of this compound: in oral administration MEI practically doesn't absorb in the gastrointestinal tract, unlike indomethacin. Thus, in order to achieve a systemic anti-inflammatory effect, special pharmaceutical compositions must be developed, which would ensure the bioavailability of MEI, reaching effective concentrations of indomethacin in the blood.

### Summary of the invention

The goal of the present invention is to create pharmaceutical compositions, based on MEI, which would ensure its bioavailability and reaching effective blood concentrations of indomethacin, providing anti-inflammatory activity in the absence (or low severity) of adverse ulcerogenic action, which would allow to use pharmaceutical compositions based on MEI as effective and safe NSAIDs with systemic action.

The goal is achieved by creating pharmaceutical compositions based on MEI, which ensure its high bioavailability and anti-inflammatory activity in the absence of adverse ulcerogenic action. These pharmaceutical compositions comprise MEI (5-methyl-2-(propan-2-yl)cyclohexan-1-yl}{[2-methyl-5-methoxy-7-(4-chlorobenzoyl)-1H-indol-3-yl]acetate}) and different pharmaceutically acceptable excipients.

In one embodiment, the pharmaceutical composition comprises a liposome-encapsulated MEI (liposomes, loaded with MEI). Lipids, preferably phospholipids, such as lecithin, for example, a hydrogenated soy lecithin powder or liquid lecithin, were used as the structure-forming component of liposomes in order to produce the liposome-encapsulated MEI. When using lecithin, addition of solubilizers was required, for example, glycerin, ethyl alcohol or other pharmaceutically acceptable solubilizer. Pharmaceutically acceptable nonpolar solvents, such as triglycerides, including medium-chain triglycerides of caprylic and capric acid, were used as a solvent (molecular vehicle) for MEI. In order to produce MEI-loaded liposomes, hydrophobic antioxidants, such as ascorbyl palmitate, osmotic agents, such as maltodextrin and other pharmaceutically acceptable ingredients, such as tocopheryl acetate, retinyl palmitate, red palm oil, Coenzyme Q, and other ingredients, required to produce a stable pharmaceutical composition based on liposome-encapsulated MEI, are added.

In other embodiments, the pharmaceutical composition is a solution or a dispersed system based on MEI and excipients: nonpolar solvents, such as mono-, di- and triglycerides of fatty acids and other pharmaceutically acceptable nonpolar solvents; alcohols, water and other polar solvents; emulsifiers, surfactants, such as lecithins, polysorbates and other pharmaceutically acceptable excipients, required for producing a stable dispersed system or a solution that would transform into a stable dispersed system in an aqueous solution and in biological medium.

MEI-based pharmaceutical compositions can also be suppositories, for example, rectal suppositories, using polyethylene glycols, such as PEG 400, PEG (Macrogol) 1500, 4000 or 6000, or solid fats (triglycerides of fatty acids) such as witepsol or supposyr, as a base for suppository formation.

MEI-based pharmaceutical compositions can be included in dosage forms for enteral use, including for oral and rectal administration, or for parenteral use, including administration onto the skin and mucous membranes of the organism, or by injections, for example, intravenous, subcutaneous, intramuscular, intra-articular injections or other methods ensuring a systemic action, and also for topical use, with the exception of eye drops and other ophthalmic compositions. MEI-based pharmaceutical compositions can be included in liquid dosage forms, including solutions, suspensions and emulsions; soft dosage forms, including salves; suppositories and patches; solid dosage forms, including tablets and capsules, and other dosage forms, ensuring the delivery of MEI and the product of its metabolism, indomethacin, to the inflammation site for treating acute and chronic inflammatory diseases.

The produced MEI-based pharmaceutical compositions were studied using in vitro and in vivo models to assess their bioavailability and pharmacokinetic profile in the blood, and also to assess the anti-inflammatory activity and adverse ulcerogenic action.

Example 1

The following components (calculated for 1 therapeutic dose of MEI) were used to produce a pharmaceutical composition, an emulsion based on liposome-encapsulated MEI:

1) MEI substance (powder) - from 10 to 100 mg;
2) Lecithin 60% - from 10 to 100 mg;
Other ingredients in amounts required for producing a stable liposomal emulsion, including:
3) Ethyl alcohol 96%;
4) Pharmaceutical glycerin;
5) Maltodextrin powder;
6) Carrageenan GS;
7) Potassium sorbate;
8) Sodium benzoate;
9) «Grindox» antioxidant;
10) Purified water.

Since MEI is lipophilic, MEI was combined with the phospholipid by fusing it with lecithin/MEI ratio of 0.5/1 to 10/1, preferably at lecithin/MEI ratio of 2/1. At this ratio the produced liposomes remain stable when stored in a gel, and high concentration of active agent is achieved in liposomal membrane and in the finished liposomal gel dosage form. The fusion temperature of lecithin and MEI substance was selected based on the physical properties of MEI and was 75±5°C. At this temperature the mixture has the required flowability and viscosity to produce proliposome bilayers with their subsequent closing on themselves, producing spherical vesicles. In this process MEI is present in the melt in its molecular form, guaranteeing its uniform distribution inside the liposomal bilayer. The proliposomes were prepared by mixing the molten lecithin/MEI mixture with an alcohol-glycerin mixture at 2/1 ratio and at a temperature of 25-55 °C, preferably at 40±5 °C. The liposomal emulsion was prepared at the same temperature as the proliposomes, preferably at 40±5 °C by homogenizing at a rate of 1000-10000 RPM, preferably at 2000-3000 RPM, for 1-10 min, preferably for 1-2 min. The liposomal emulsion was stabilized by a carrageenan mixture (carrageenan GS) at a temperature of 75 °C.

Example 2

The following components (calculated for 1 therapeutic dose of MEI) were used to produce a powdered pharmaceutical composition based on liposome-encapsulated MEI:

1) MEI substance (powder) - from 10 to 100 mg;
2) Hydrogenated lecithin (powder) - from 10 to 100 mg;
3) Maltodextrin - from 20 to 100 mg;
4) Medium-chain triglycerides of caprylic and capric acid (MCTG) - from 50 to 200 mg;
Other ingredients in amounts, required for producing a stable liposomal emulsion, including:

5) «Grindox» antioxidant;
6) Purified water.

Hydrogenated lecithin and maltodextrtin were mixed at a ratio of 5/1 to 1/5, preferably at 1/1, and dissolved in purified water, producing a viscous mass containing phospholipid bilayers, then at a temperature of at least 50 °C a mixture of fat-soluble components (molten MEI in a molecular vehicle MCTG) and the remaining amount of purified water was added. MEI melt in MCTG was produced at a MCTG/MEI ratio of 0.5/1 to 10/1, preferably at 1/1. The temperature 75 ± 5 °C was used, at this temperature MEI is present in the melt in its molecular form, guaranteeing its uniform distribution inside the liposomal bilayer, while the melt has the required flowability for its administration into proliposomes. «Grindox» antioxidant, containing butylhydroxytoluene, butylhydroxyanisole, ascorbyl palmitate, lecithin and rapeseed oil, was added to concentration 0.02-0.1% of the fatty mass. To produce a liposomal emulsion, the mixture with 20% concentration of dry components in water was homogenized for 1-10 min, preferably for 1-2 min, at a temperature of 35-70 °C, preferably 55±5 °C. The liposomal emulsion was filtered through a 0.16-0.2 mm nylon filter and dried using a spray dryer at a temperature of 60-80 °C, preferably 70 °C, producing a finely dispersed powder with moisture content no more than 5%.

Example 3

The following components were used to produce a MEI-based pharmaceutical composition, a solution in triglycerides producing an emulsion in aqueous medium:

1) MEI substance (powder) - from 10 to 100 mg;
2) Lipoid C 80 - from 20 to 100 mg;
3) Polyoxyethylene sorbitan monooleate (Tween 80) - from 1 to 20 mg;
4) Medium-chain triglycerides (Miglyol 812 N) - from 200 to 1000 mg.

Pre-weighted Miglyol 812 N was put into a 100 ml vial, put onto a water bath, the stirrer was turned on, and Miglyol was heated to 40-50 °C. Upon reaching the temperature of 40-50 °C, finely ground and pre-weighted Lipoid C 80 was added while stirring, the mixture was stirred for 30 min until the complete dissolution of Lipoid C 80.
Then, at 40-50 °C pre-weighted MEI substance was put in in small portions while stirring constantly at a Lipoid/MEI ratio of 1/2 to 2/1, preferably at 1/2 to 1/1, and stirred for 15 min until the complete dissolution of the substance. After producing a homogenous solution, pre-weighted Tween 80 is added while stirring constantly at 40-50 °C and stirred for 5 min. After stirring, the remaining Miglyol 812 N was added at 40-50 °C and stirred for 10 min until the solution became clear and homogenous. Good solubility of MEI in Miglyol 812 N and a combined addition of two surfactants, Lipoid C 80 and polyoxyethylene sorbitan monooleate, ensures the formation of a stable emulsion when the composition gets into the stomach, increasing the bioavailability of MEI.

Example 4

The following components were used to produce a MEI-based pharmaceutical composition, a solution in mono- and diglycerides of fatty acids, producing an emulsion in aqueous medium:

1) MEI substance (powder) - from 10 to 100 mg;
2) Polysorbate 20 NF - from 5 to 15 mg;
3) Food-grade mono- and diglycerides of fatty acids (E471) - from 200 to 1000 mg.

Pre-weighted E471 was put into a 100 ml vial, put onto a water bath, the stirrer was turned on and E471 was heated to 40-50 °C. Then, at 40-50 °C pre-weighted MEI substance was put in in small portions while stirring constantly at a E471/MEI ratio of 1/1 to 20/1, preferably at 5/1 to 10/1, and stirred for 20 min until the complete dissolution of the substance. After producing a homogenous solution, pre-weighted Polysorbate 20 NF was added in amount of 2-15% of the mixture while stirring constantly at 40 °C and stirred for 10-15 min until the solution became clear and homogenous.
The resulting pharmaceutical composition makes a stable emulsion when mixed with water or stomach contents.

Example 5

The following components (calculated for 1 suppository containing 1 therapeutic dose of MEI) were used to produce a MEI-based pharmaceutical composition, rectal suppositories:

1) MEI substance (powder) - from 10 to 100 mg;
2) Dimethyl sulfoxide (DMSO) - from 200 to 400 mg;
3) Tween 80 - from 5 to 20 mg;
4) Benzyl alcohol - from 5 to 20 mg;
5) Purified water - from 3 to 10 mg;
6) Suppository base - from 1.3 to 2.36 g.

The suppositories have the following physical-chemical characteristics: color - light yellow, melting point - no higher than 35.5°C.

Example 6

The following components (calculated for 1 g of salve) were used to produce a MEI-based pharmaceutical composition, a salve:

1) MEI substance (powder) - from 25 to 100 mg;
2) Dimethyl sulfoxide (DMSO) - from 150 to 300 mg;
3) Polyethylene glycol (PEG 400) - from 20 to 100 mg;

Salve base:

4) Polyethylene glycol (PEG 4000) - from 200 to 300 mg;
5) Propylene glycol - from 100 to 200 mg.

The salve base was put into a mortar and melted on an ultrasound bath while heating. The substance was put into a mortar, DMSO and PEG 400 were added, mixed in the mortar until the complete dissolution of the substance, then the salve base was added and mixed until the salve became homogenous. The resulting salve with 2.5-10 % MEI content didn't separate at 60 °C for 30 min.

MEI is metabolized in the organism, producing its active metabolite indomethacin, which has potent anti-inflammatory action and at the same time has potent adverse ulcerogenic action. However, MEI is almost non-bioavailable without special excipients (such as a suspension in starch solution or carboxymethyl cellulose), it doesn't absorb into the blood and can't have systemic action. Therefore, it was important to determine the pharmacokinetic properties of MEI-based compositions, in particular, to determine indomethacin concentration in the blood after oral administration of MEI-based compositions.

Example 7

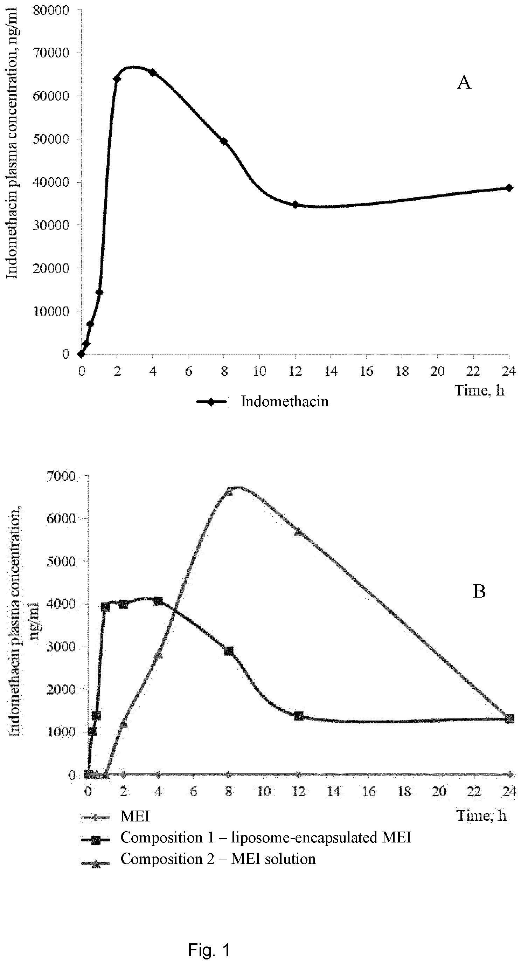

Pharmacokinetic properties of two MEI-based compositions were studied: composition 1 (liposome-encapsulated MEI), produced as described in example 1, and composition 3 (MEI solution), produced as described in example 3. MEI substance (as a suspension in starch solution) and indomethacin were used as reference drugs. Rats of Sprague Dawley stock were used. All test compounds were administered orally to rats in 25 mg/kg dose. Animal blood was sampled at certain time points (0; 0.25; 0.5; 1, 2, 4, 8, 16 and 24 hours) for analysis. Indomethacin content in animal blood plasma was determined using high-performance liquid chromatography (HPLC) method. Pharmacokinetic profiles of indomethacin in blood plasma after test compound administration are presented in Figure 1.

The data shows that after oral administration of indomethacin in 25 mg/kg dose its maximal concentration in rat blood plasma ($C_{max}$) was 65496 ng/ml, time of reaching the maximal concentration ($T_{max}$) was 4 hours (Figure 1 A). After the administration of MEI substance (as a suspension in starch solution) in 25 mg/kg dose, indomethacin wasn't detected in blood plasma in any time point, its concentration was below the detection limit, i.e. MEI in such form is practically non-bioavailable and can't exert its anti-inflammatory action. After the administration of MEI-based pharmaceutical compositions 1 and 3 in 25 mg/kg dose $C_{max}$ of indomethacin was 6647 and 4061 ng/ml, respectively, $T_{max}$ was 8 and 4 h, respectively (Figure 1 B). Thus, MEI is bioavailable when administered in special compositions (in liposomes or solution). At the same time, maximal indomethacin concentration in blood plasma after the administration of MEI-based compositions is about 10-15 lower than after the administration of indomethacin, allowing to reduce the adverse systemic toxic effects, for example, ulcerogenic action.

Example 8

Anti-inflammatory activity of MEI-based pharmaceutical compositions compared to MEI substance (as a suspension in starch solution) and indomethacin after oral administration was studied in a model of acute inflammation - paw edema induced by inflammation mediator serotonin. Rats of Sprague Dawley stock were used. Inflammation was induced by a subplantar administration of serotonin (0.1 mg/ml) in one hind paw, and saline in the other hind paw (the control paw). Test compounds were administered orally 2 hours before the serotonin administration. Inflammation severity was measured 30 min after serotonin administration using a plethysmometer (UGO BASILE, Italy). Inflammation severity was measured by plethyosmography as the difference between the masses of control and test paws, expressed in ml. Anti-inflammatory activity of the test compounds was assessed by the inhibition index, calculated using the following formula:

$$II = ((S_{ct} - S_t)/ S_{ct}) \times 100\% ,$$

where II - inflammation inhibition index in %;
$S_{ct}$ - severity of inflammation in the control group, ml;
St - severity of inflammation in the test group, ml.

The study results are presented in Table 1.

Table 1 - Anti-inflammatory activity of MEI-based pharmaceutical compositions in a model of serotonin-induced edema (inflammation inhibition index, %, n = 8)

| Group | Dose of active agent, mg/kg | Inflammation inhibition index, % | Significance of difference from control, p |
|---|---|---|---|
| Control | 0 | - | - |
| MEI | 25 | 4.3 | > 0.5 |
| Composition 1 (liposome-encapsulated MEI) | 25 | 38.8 | < 0.001 |
| Composition 3 (MEI solution) | 25 | 33.4 | < 0.01 |
| Indomethacin | 10 | 43.5 | < 0.01 |

The results show that MEI (as a suspension of the substance in starch solution) after oral administration doesn't have significant anti-inflammatory effects in a model of acute inflammation, while MEI-based pharmaceutical compositions after oral administration in 25 mg/kg dose have potent anti-inflammatory action, being only slightly weaker than the reference drug indomethacin. Thus, the developed compositions ensure the bioavailability of MEI and its systemic anti-inflammatory action.

Example 9

Anti-inflammatory and analgesic activity of MEI-based pharmaceutical composition after oral administration was studied in a model of chronic inflammation - adjuvant-induced rheumatoid arthritis in rats. To induce arthritis the rats were administered 100 mcl of complete Freund adjuvant (CFA), supplied by Sigma Aldrich, into the plantar surface of right hind paw. The control (intact) group 1 has received 100 mcl of saline. After CFA injection, local inflammation develops in a few hours, with subsequent pathologic changes in metacarpophalangeal joints and distal interphalangeal joints. Test compound (composition 3 - MEI solution) and the reference drug (indomethacin) were administered once daily orally for 22 days, starting from 7th day after pathology induction. Test compound was administered in 6.25, 12.5 and 25 mg/kg doses. The reference drug indomethacin was administered in 1 mg/kg dose (in higher dose of 5 mg/kg animal mortality is observed). To assess the anti-inflammatory action of MEI in animals by pelthysmography, inflammation severity was measured as the difference between the masses of control and test paws, expressed in ml. Analgesic activity of MEI in animals was assessed by tactile reactivity threshold using the method of Chaplan et al. [5] using the kit of 8 standard von Frey hairs (Stoelting, USA).
The results of the experiment are presented in Tables 2 and 3.

Table 2 - Anti-inflammatory activity of MEI-based pharmaceutical composition in a model of adjuvant-induced rheumatoid arthritis (inflammation severity, ml, X±SE, n=8)

| Group | Test compound dose, mg/kg | Experiment day | | | |
|---|---|---|---|---|---|
| | | 7** | 14 | 21 | 28 |
| Control 1 (intact animals) | 0 | 0±0.03 | 0±0.03 | 0±0.04 | 0±0.04 |
| Control 2 (untreated pathology) | 0 | 3.0±0.61 | 4.3±0.59 | 3.9±0.48 | 3.6±0.45 |
| MEI | 6.25 | 2.8±0.54 | 2.5±0.76* | 1.8±0.49* | 1.9±0.51* |
| | 12.5 | 2.6±0.38 | 1.9±0.37* | 2.0±0.40* | 1.1±0.21* |
| | 25 | 2.4±0.43 | 1.5±0.22* | 0.9±0.07* | 0.8±0.12* |
| Indomethacin | 1 | 3.0±0.59 | 1.8±0.24* | 1.5±0.18* | 1.4±0.28* |

Notes:
1 - * - differences from control group 2 were statistically significant in the examination day, Fisher criterion (LSD test), p<0,05;
2 - ** - testing on Day 7 was performed before test compound administration.

Table 3 - Analgesic activity of MEI-based pharmaceutical composition in a model of adjuvant-induced rheumatoid arthritis (median-effective tactile reactivity threshold in an injected limb, g, X±SE, n=8)

| Group | Test compound dose, mg/kg | Experiment day | | |
|---|---|---|---|---|
| | | 7** | 21 | 28 |
| Control 1 (intact animals) | 0 | 25.7±1.17 | 25.6±1.75 | 24.8±1.72 |
| Control 2 (untreated pathology) | 0 | 11.5±2.92 | 6.5±1.65 | 8.9±1.98 |
| MEI | 6.25 | 11.7±2.93 | 10.4±3.09 | 9.6±2.67 |
| | 12.5 | 11.8±3.12 | 13.3±2.55* | 14.8±2.35* |
| | 25 | 9.8±2.14 | 16.6±2.64* | 18.2±2.57* |
| Indomethacin | 1 | 9.9±2.34 | 14.5±3.18* | 16.0±3.04* |

Notes:
1 - * - differences from control group 2 were statistically significant in the examination day, Fisher criterion (LSD test), p<0,05;
2 - ** - testing on Day 7 was performed before test compound administration.

The results demonstrate that MEI-based pharmaceutical composition in all tested doses (from 6.25 to 25 mg/kg) exert potent anti-inflammatory action, and in 12.5 and 25 mg/kg doses exert potent analgesic action in a model of chronic inflammation - adjuvant-induced rheumatoid arthritis, being no less effective than the reference drug indomethacin.

Example 10

Anti-inflammatory activity of MEI-based pharmaceutical composition after parenteral administration was studied in a model of carrageenan-induced edema [6]. Rats of Sprague Dawley stock were used. 50 mcl of saline ("sodium chloride 0.9% solution for infusion", NPK ESCOM, Russia) was injected intraplantarly into one hind paw (control paw), while 50 mcl of 1% λ-carrageenan solution was injected into another paw.
Inflammation severity was measured after 4 h using a plethysmometer (IITC Life Science, USA). Inflammation severity was measured by plethyosmography as the difference between the masses of control and test paws, expressed in ml. Test compound, pharmaceutical composition №4 (MEI solution) was administered parenterally (intraperitoneally) 1 hour before λ-carrageenan administration, animals receiving the same amount of the vehicle were used as control. The animals were deprived of food 24 h before the study, water access was not limited.

The results of the experiment are presented in Table 4.

Table 4 - Anti-inflammatory activity of MEI-based pharmaceutical composition after intraperitoneal administration in a model of carrageenan-induced edema in rats (X±SE, n=7)

| Group | Dose, mg/kg | Edema size: | |
|---|---|---|---|
| | | ml | % from control |
| Control | 0 | 1.109±0.090 | 100.0 |
| MEI | 0.1 | 1.011±0.115 | 91.2 |
| | 0.5 | 0.675±0.065* | 60.9 |
| | 1.0 | 0.552±0.097* | 45.0 |
| | 10 | 0.201±0.176* | 18.1 |
| | 50 | 0.288±0.152* | 26.0 |
| Note - *differences from control are statistically significant, $p < 0,05$. | | | |

The results demonstrate that MEI-based pharmaceutical composition in parenteral (intraperitoneal) administration in doses from 0.5 to 50 mg/kg has potent anti-inflammatory action in a model of acute inflammation.

Example 11

Local anti-inflammatory activity of MEI-based pharmaceutical composition, a salve, after topical administration was studied in a model of carrageenan-induced edema. Rats of Sprague Dawley stock were used. Carrageenan-induced edema was produced as described in Example 10. Test compound, MEI-based pharmaceutical composition as a 10% salve, was applied at rat hind paw surface immediately after carrageenan administration and then an additional three times with 1 hour between applications. After test compound application the animal was left immobilized for 4-5 min, then returned to the cage. Salve base without MEI was applied to control group animals. The animals were deprived of food 24 h before the study, water access was not limited.

The results of the experiment are presented in Table 5.

Table 5 - Anti-inflammatory activity of MEI-based pharmaceutical composition (10% salve) in a model of carrageenan-induced edema in rats (X±SE, n=8)

| Group | Edema size: | |
|---|---|---|
| | ml | % from control |
| Control | 0.993 ± 0.151 | 100.0 |
| MEI (10% salve) | 0.527 ± 0.075* | 53.0 |
| Note - *differences from control are statistically significant, $p < 0,05$. | | |

The results demonstrate that MEI-based pharmaceutical composition in topical administration as a salve containing 10% MEI has potent local anti-inflammatory action in a model of acute inflammation.

Example 12

Antipyretic activity of MEI-based pharmaceutical composition was studied in a model of yeast-induced fever in rats. Rats of Sprague Dawley stock were used. Fever was induced in rats by subcutaneous administration of baker's yeast suspension in 2000 mg/kg dose (calculated for dry yeast basis) in 2 ml volume into the animal withers 18 hours before test compound administration. Animal body temperature was measured using an electronic rectal thermometer (MLT1403 Rectal Temperature Probe and PowerLab 4/35 4-channel registration system module, ADInstruments PtyLtd, Australia). Temperature was measured before yeast administration and 18 h after yeast administration. Afterwards the animals were administered the test compound, MEI-based pharmaceutical composition (composition 3 - MEI solution,

8

produced as described in Example 3) in 25, 50 and 100 mg/kg doses. Indomethacin in 10 mg/kg dose was used as a reference drug. Animals in control group have received the vehicle. Temperature was measured 2 and 4 hours after test compound administration.

The results of the experiment are presented in Table 6.

Table 6 - Antipyretic activity of MEI-based pharmaceutical composition in a model of yeast-induced fever in rats (n=6)

| Group | Dose of active agent, mg/kg | Average body temperature 2 h after test compound administration, °C | Average body temperature 4 h after test compound administration, °C |
|---|---|---|---|
| Control | 0 | -0.1 | +0.15 |
| MEI | 25 | -0.53 | -0.45 |
|  | 50 | -0.93 | -0.82 |
|  | 100 | -0.78 | -0.68 |
| Indomethacin | 10 | -0.98 | -0.85 |

The results demonstrate that MEI-based pharmaceutical composition, a solution, in oral administration has potent antipyretic action in a model of yeast-induced fever.

Example 13

To assess the severity of adverse ulcerogenic action the test compound (composition 1 - MEI solution) and the reference drug (indomethacin) were administered 4 times orally to rats. 3 hours after the last administration the animals were sacrificed, their stomachs extracted, dissected at the least curve line and washed with saline to remove the contents. Ulcerogenic action was measured using the following 4-point scale:

0 - no damage;
0.5 - slight hyperemia of gastric mucosa;
1 - weak localized damage of gastric mucosa (single point bleeding, severe hyperemia);
2 - weak distributed damage of gastric mucosa (small ulcers, erosions, multiple bleeding);
3 - severe and distributed damage of gastric mucosa (erosions, ulcers, bleeding);
4 - gross damage of the whole stomach (massive bleeding, erosion, ulcers, perforation).

The results of the experiment are presented in Table 7.

Table 7 - Severity of ulcerogenic action of MEI-based pharmaceutical composition

| Group | Dose of active agent, mg/kg | Average number of erosions | Average number of ulcers | Average score |
|---|---|---|---|---|
| Control | 0 | 0 | 0 | 0 |
| MEI | 25 | 0 | 0 | 0 |
|  | 50 | 0 | 0 | 0 |
|  | 100 | 0 | 0.8 | 0.4 |
| Indomethacin | 5 | 3.2 | 8 | 3 |

The results demonstrate that animals receiving four 5 mg/kg doses of indomethacin had multiple ulcers and erosions, while animals receiving MEI-based pharmaceutical composition in 25 and 50 mg/kg doses had no gastric mucosa disorders, while animals receiving MEI-based pharmaceutical composition in maximal 100 mg/kg dose had only weak point damage. Therefore, the tested MEI-based pharmaceutical composition in fourfold administration in the studied doses doesn't have ulcerogenic action, unlike indomethacin.

The abovementioned examples demonstrate that enteral, parenteral and topical administration of the disclosed pharmaceutical compositions, based on a menthyl ester of indomethacin, allows to achieve effective concentrations of indomethacin, sufficient fohaving potent anti-inflammatory activity, but without negative adverse ulcerogenic action.

Thanks to this, the disclosed MEI-based pharmaceutical compositions can be used as effective and safe NSAIDs.

**References**

1. US Patent No. 6,306,890.
2. US Patent No. 6,762,182.
3. US Patent No. 8,097,646.
4. Sawraj Sh., Bhardawaj T.R., Sharma P.D Design, synthesis, and evaluation of novel indomethacin-antioxidant codrugs as gastrosparing NSAIDs. Med Chem Res (2012) 21:834-843 DOI 10.1007/s00044-011-9589-1.
5. Chaplan, S. R., et al. Quantitative assessment of tactile allodynia in the rat paw. // J.Neurosci.Methods. 1994. Vol. 53.P. 55-63.
6. Methodical recommendations for preclinical research of non-steroidal anti-inflammatory drugs. / Guideline on preclinical research of drugs. Moscow: Grif and Co. Part 1. 2012. P. 748-760.

**Claims**

1. The use of a pharmaceutical composition, having anti-inflammatory, analgesic and antipyretic action, based on a menthyl ester of indomethacin, comprising 5-methyl-2-(propan-2-yl)cyclohexan-1-yl]{[2-methyl-5-methoxy-7-(4-chlorobenzoyl)-1H-indol-3-yl]acetate} and pharmaceutically acceptable excipients, ensuring the bioavailability of the menthyl ester of indomethacin, as an anti-inflammatory drug for treating acute and chronic inflammatory diseases by its enteral administration to a mammal, including oral and rectal administraiton, or by its parenteral administration, including by injections or application onto the skin.

Fig. 1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6306890 B
- US 6762182 B
- US 8097646 B

**Non-patent literature cited in the description**

- **SAWRAJ SH. ; BHARDAWAJ T.R. ; SHARMA P.D.** Design, synthesis, and evaluation of novel indomethacin-antioxidant codrugs as gastrosparing NSAIDs. *Med Chem Res,* 2012, vol. 21, 834-843
- **CHAPLAN, S. R. et al.** Quantitative assessment of tactile allodynia in the rat paw. *J.Neurosci.Methods,* 1994, vol. 53, 55-63
- Methodical recommendations for preclinical research of non-steroidal anti-inflammatory drugs. / Guideline on preclinical research of drugs. *Moscow: Grif and Co,* 2012, 748-760